# EUROPÄISCHE PATENTSCHRIFT

(11) **EP 2 787 903 B1**
(45) Veröffentlichungstag und Bekanntmachung des Hinweises auf die Patenterteilung: **08.05.2019**
(21) Anmeldenummer: 12791740.9
(22) Anmeldetag: 17.11.2012
(51) Int. Cl.: A61B 17/29, A61B 17/16, A61B 17/00, A61B 17/28

(54) **MEDIZINISCHES INSTRUMENT**
MEDICAL INSTRUMENT
INSTRUMENT MÉDICAL

(30) Priorität: 09.12.2011 DE 102011056235
(43) Veröffentlichungstag der Anmeldung: 15.10.2014
(73) Patentinhaber: Aesculap AG, 78532 Tuttlingen (DE)
(72) Erfinder: SEEL, Wilhelm, 78333 Stockach (DE)
(74) Vertreter: Hoeger, Stellrecht & Partner Patentanwälte mbB
(86) Internationale Anmeldenummer: PCT/EP2012/072937
(87) Internationale Veröffentlichungsnummer: WO 2013/083390

(56) Entgegenhaltungen:
- WO-A1-01/35838
- WO-A1-03/094756
- DE-A1- 4 316 768
- DE-C- 356 185
- DE-U1- 29 822 166
- DE-U1-202009 002 235
- US-A- 4 043 343
- US-A- 5 507 774

## Beschreibung

Die vorliegende Erfindung betrifft ein medizinisches Instrument mit mindestens einem bewegbar angeordneten Werkzeugelement und mindestens einem um eine Schwenkachse verschwenkbar gelagerten, mit dem mindestens einen Werkzeugelement direkt oder indirekt gekoppelten Betätigungselement zum Bewegen des Werkzeugelements infolge einer Verschwenkbewegung des Betätigungselements, wobei zur verschwenkbaren Lagerung des mindestens einen Betätigungselements eine Schwenklagerung umfassend eine Lagerwellenaufnahme und eine in einer Arbeitsstellung in der Lagerwellenaufnahme gehaltene Lagerwelle vorgesehen ist, wobei das mindestens eine Betätigungselement die Lagerwelle oder die Lagerwellenaufnahme umfasst, wobei die Lagerwellenaufnahme eine Einführöffnung umfasst und dass die Lagerwelle in einer Montagestellung in einer Richtung quer oder im Wesentlichen quer zur Schwenkachse durch die Einführöffnung in die Lagerwellenaufnahme einführbar ist, wobei die Einführöffnung in Form eines die Lagerwellenaufnahme einseitig öffnenden Schlitzes ausgebildet ist, wobei sich der Schlitz in radialer Richtung von der Schwenkachse weg erstreckt, wobei der Schlitz eine Breite aufweist, welche kleiner ist als ein von der Lagerwellenaufnahme definierter Durchmesser, wobei die Lagerwelle mindestens einen zylindrischen Wandflächenabschnitt umfasst, welcher konzentrisch zur Schwenkachse ausgebildet ist.

Medizinische Instrumente mit mindestens einem bewegbar angeordneten Werkzeugelement und mindestens einem um eine Schwenkachse verschwenkbar gelagerten, mit dem mindestens einen Werkzeugelement direkt oder indirekt gekoppelten Betätigungselement zum Bewegen des Werkzeugelements infolge einer Verschwenkbewegung des Betätigungselements, wobei zur verschwenkbaren Lagerung des mindestens einen Betätigungselements eine Schwenklagerung umfassend eine Lagerwellenaufnahme und eine in einer Arbeitsstellung in der Lagerwellenaufnahme gehaltene Lagerwelle vorgesehen ist, wobei das mindestens eine Betätigungselement die Lagerwelle oder die Lagerwellenaufnahme umfasstsind in vielfältiger Form bekannt. Die Schwenklagerung eines oder mehrerer Betätigungselemente der Instrumente wird üblicherweise dadurch realisiert, dass die Lagerwellenaufnahme in Form einer Bohrung ausgebildet ist, in welche die Lagerwelle koaxial zu der von ihr definierten Längsachse eingeschoben und fixiert wird, beispielsweise durch Vernieten, Dengeln oder mittels einer Schraubverbindung. Nachteilig dabei ist jedoch der mit der Ausbildung der Schwenklagerung verbundene hohe Montageaufwand.

Instrumente der eingangs beschriebenen Art sind beispielsweise aus der DE 43 16 768 A1 bekannt. Weitere medizinische Instrumente sind in der DE 20 2009 002 235 U1, der DE 356 185 C, der US 4,043,343, der US 5,507,774, der WO 01/35838 A1, der DE 298 22 166 U1 sowie der WO 03/094756 A1 bekannt. Es ist daher eine Aufgabe der vorliegenden Erfindung, ein medizinisches Instrument der eingangs beschriebenen Art so zu verbessern, dass es besonders einfach montiert werden kann.

Die dem Anmeldungsgegenstand zugrundeliegende Aufgabe wird durch ein erfindungsgemäßes Instrument mit den im Anspruch 1 angegebenen Merkmalen gelöst, besondere Ausführungsformen sind in den Unteransprüchen 2- 15 angegeben.

Diese Aufgabe wird bei einem medizinischen Instrument der eingangs beschriebenen Art erfindungsgemäß dadurch gelöst, dass die Lagerwelle aus einem zylindrischen oder im Wesentlichen zylindrischen Grundkörper gebildet ist und zwei von der Schwenkachse beabstandete seitliche Abflachungen aufweist und dass die Lagerwelle einstückig mit dem Instrument oder dem mindestens einen Betätigungselement ausgebildet ist.

Die erfindungsgemäß vorgeschlagene Weiterbildung sieht insbesondere vor, die Lagerwelle am mindestens einen Betätigungselement oder am Instrument anzuordnen oder auszubilden, und zwar einstückig mit dem Instrument oder dem mindestens einen Betätigungselement auszubilden, und zur Montage des Instruments die Lagerwelle einfach durch die Einführöffnung hindurch in die Lagerwellenaufnahmen einzuführen. Weitere Elemente, Werkzeuge oder Bearbeitungsschritte, wie sie bei bekannten Instrumenten erforderlich sind, um einen Schluss zweier relativ zueinander verschwenkbarer Teil des Instruments auszubilden, sind dann für die Montage des mindestens einen Betätigungselements am Instrument nicht erforderlich. Nach Einführen der Lagerwelle in die Lagerwellenaufnahme kann das mindestens eine Betätigungselement insbesondere direkt um die Schwenkachse verschwenkt werden, um infolge einer solchen Verschwenkbewegung das mindestens eine Werkzeugelement direkt oder indirekt in gewünschter Weise zu bewegen. Besonders einfach ausbilden lässt sich das medizinische Instrument dadurch, dass die Einführöffnung in Form eines die Lagerwellenaufnahme einseitig öffnenden Schlitzes ausgebildet ist. Die Lagerwelle kann so durch den Schlitz in die Lagerwellenaufnahme in einer Richtung quer zur Schwenkachse eingeführt werden. Besonders einfach und direkt einführen lässt sich die Lagerwelle in die Lagerwellenaufnahmen dadurch, dass sich der Schlitz in radialer Richtung von der Schwenkachse weg erstreckt. Vorteilhaft ist es, dass der Schlitz eine Breite aufweist, welche kleiner ist als ein von der Lagerwellenaufnahme definierter Durchmesser. Ein solcher Schlitz ermöglicht es, eine entsprechend geformte Lagerwelle sicher und unverlierbar in der Lagerwellenaufnahme zu halten, wenn das Instrument nicht die Montagestellung einnimmt. Nur in der Montagestellung lässt sich so die Lagerwelle durch den Schlitz in die Lagerwellenaufnahme ein- oder wieder aus dieser herausführen. Vorteilhaft ist es, dass die Lagerwelle mindestens einen zylindrischen Wandflächenabschnitt umfasst, welcher konzentrisch zur Schwenkachse ausgebildet ist. Günstig ist es, dass zwei derartige Wandflächenabschnitte vorgesehen sind. Zylindrische Wandflächenabschnitte können insbesondere im Zusammenwirken mit einem oder mehreren korrespondierenden hohlzylindrischen Innenwandflächenabschnitten der Lagerwellenaufnahme eine definierte Verschwenkbewegung der Lagerwelle in der Lagerwellenaufnahme führen. Besonders einfach herstellen lässt sich die Lagerwelle dadurch, dass sie aus einem zylindrischen oder im Wesentlichen zylindrischen Grundkörper gebildet ist und zwei von der Schwenkachse beabstandete seitliche Abflachungen aufweist. Abflachungen lassen sich auf einfache Weise durch Fräsen, Feilen oder andere spanende Bearbeitung des Grundkörpers herstellen. Insbesondere kann die Lagerwelle einstückig mit dem Instrument oder dem mindestens einen Betätigungselement ausgebildet werden.

Vorteilhaft ist es, wenn die in die Lagerwellenaufnahme eingeführte Lagerwelle durch Verschwenken um die Schwenkachse von der Montagestellung in die Arbeitsstellung bringbar ist, in welcher die Lagerwelle in der Lagerwellenaufnahme unverlierbar gehalten ist. Dies gestattet es, die speziell ausgebildete Lagerwellenaufnahme in Verbindung mit der Lagerwelle insbesondere so zu gestalten, dass zunächst durch eine rein translatorische Bewegung die Lagerwelle in die Lagerwellenaufnahme hinein bewegbar und durch anschließende Verschwenkbewegung in die Arbeitsstellung bringbar ist, in welcher die Lagerwelle unverlierbar in der Lagerwellenaufnahme gehalten ist, also in einer Stellung, in welcher sie nicht durch eine translatorische Bewegung wieder zurück durch die Einführöffnung aus der Lagerwellenaufnahme herausbewegt werden kann.

Günstig ist es, wenn der Schlitz aufeinander zu weisende Innenflächen umfasst. Diese können so auf einfache Weise eine Breite des Schlitzes vorgeben.

Besonders einfach herstellen lässt sich die Einführöffnung, wenn die Innenflächen parallel oder im Wesentlichen parallel zu einer die Schwenkachse enthaltenden Ebene verlaufen. Sie können dann beispielsweise durch einen Sägeschnitt oder durch Fräsen beim Ausbilden des Schlitzes präpariert werden.

Günstig ist es, wenn die Lagerwellenaufnahme mindestens einen hohlzylindrischen Innenwandflächenabschnitt umfasst, welcher konzentrisch zur Schwenkachse ausgebildet ist. Ein derartig geformter Innenwandflächenabschnitt ermöglicht es, eine Schwenkbewegung der Lagerwelle und damit beispielsweise des mindestens einen Betätigungselements definiert um die Schwenkachse zu führen.

Gemäß einer weiteren vorteilhaften Ausführungsform der Erfindung kann vorgesehen sein, dass ein vom mindestens einen zylindrischen Wandflächenabschnitt definierter Außendurchmesser der Lagerwelle und ein vom mindestens einen Innenwandflächenabschnitt definierter Innendurchmesser der Lagerwellenaufnahme aneinander angepasst sind für ein spielfreies oder im Wesentlichen spielfreies Verschwenken der Lagerwelle in der Lagerwellenaufnahme. Diese Ausgestaltung ermöglicht eine definierte Verschwenkung des mindestens einen Betätigungselements um die Schwenkachse.

Günstigerweise verlaufen die Abflachungen parallel zueinander. Dies ermöglicht es insbesondere, die Lagerwelle in definierter Weise durch einen die Lagerwellenaufnahme seitlich öffnenden Schlitz in diese einzuführen, welcher vorteilhafterweise parallel zueinander verlaufende Innenflächen umfassen kann.

Vorteilhaft ist es, wenn ein Abstand der Abflachungen voneinander kleiner ist als eine Breite der Einführöffnung. So ist es möglich, die Lagerwelle definiert und ohne zu Verklemmen durch die Einführöffnung hindurch in die Lagerwellenaufnahme einzuführen.

Günstig ist es, wenn das Instrument eine Betätigungseinrichtung umfasst, welche das mindestens eine Betätigungselement und zweites, am Instrument unbeweglich angeordnetes Betätigungselement umfasst. Eine solche Betätigungseinrichtung ermöglicht es insbesondere, das Instrument einerseits sicher zu halten und andererseits das mindestens eine Werkzeugelement definiert und gefühlvoll zu bewegen durch entsprechende Betätigung des mindestens einen Betätigungselements.

Vorteilhaft ist es, wenn die Lagerwellenaufnahme einen Anschlag umfasst zum Begrenzen einer Schwenkbewegung des Betätigungselements in einer Arbeitsstellung, in welcher die Lagerwelle in der Lagerwellenaufnahme unverlierbar gehalten ist. Durch den Anschlag kann eine Schwenkbewegung des mindestens einen Betätigungselements am Instrument auf einfache und sichere Weise begrenzt werden.

Besonders einfach ausbilden lässt sich der Anschlag, wenn er in Form eines an der Lagerwellenaufnahme ausgebildeten Vorsprungs ausgebildet ist. Insbesondere kann dieser einstückig mit der Lagerwellenaufnahme ausgebildet sein.

Vorteilhaft ist es, wenn der Anschlag diejenige Stellung der Betätigungselemente definiert, in welcher sie maximal voneinander weg oder maximal aufeinander zu verschwenkt sind. So lassen sich auf einfache Weise insbesondere extreme Stellungen der Betätigungselemente relativ zueinander definieren und eine Beweglichkeit derselben relativ zueinander einschränken.

Günstig ist es, wenn der Anschlag eine Anschlagfläche umfasst, an welcher eine Abflachung der Lagerwelle in derjenigen Stellung, in welcher die Betätigungselemente maximal voneinander weg oder aufeinander zu verschwenkt sind, mindestens teilweise flächig anliegt. So kann beim Anschlagen der Lagerwelle am Anschlag insbesondere eine möglichst geringe Flächenpressung erreicht und dadurch eine Standzeit des Instruments maximiert werden. Gemäß einer vorteilhaften Ausführungsform der Erfindung kann vorgesehen sein, dass das mindestens eine Betätigungselement in Form einer verschwenkbaren Branche ausgebildet ist, welche ein erstes Koppelelement umfasst, welches mit einem zweiten Koppelelement, welches am mindestens einen Werkzeugelement oder an einem mit diesem gekoppelten Kraftübertragungsglied angeordnet oder ausgebildet ist, in einer Koppelstellung in Eingriff bringbar ist. Die ersten und zweiten Koppelelemente dienen also insbesondere dazu, die auf das mindestens eine Betätigungselement eingeleitete Kraft direkt oder indirekt auf das mindestens eine Werkzeugelement zu übertragen. Hierzu können die Koppelelemente insbesondere kraft- und/oder formschlüssig miteinander in Eingriff stehen, wobei sie vorteilhafterweise eine Bewegung, insbesondere eine Translations- und/oder Schwenkbewegung, relativ zueinander ermöglichen.

Günstig ist es, wenn das Instrument eine Rückstelleinrichtung zum automatischen Überführen des mindestens einen Betätigungselements von einer aus einer Grundstellung ausgelenkten Stellung zurück in die Grundstellung umfasst, wobei die Grundstellung diejenige Arbeitsstellung definiert, in welcher das mindestens eine Betätigungselement gegenüber der Montagestellung um den größten Schwenkwinkel gedreht ist. Durch die Rückstelleinrichtung kann somit insbesondere verhindert werden, dass das Instrument ohne Einwirkung äußerer Kräfte, also wenn es unbetätigt ist, in die Montagestellung überführt werden kann, in welcher sich die Lagerwelle aus der Lagerwellenaufnahme entnehmen lässt. Die Rückstelleinrichtung bildet somit quasi eine Sicherungseinrichtung des Instruments, um ein unbeabsichtigtes Zerlegen desselben zu vermeiden. Insbesondere kann sie derart ausgebildet sein, dass in der Montagestellung die Betätigungselemente der Betätigungseinrichtung entweder maximal aufeinander zu oder maximal voneinander weg verschwenkt sind.

Besonders einfach ausbilden lässt sich die Rückstelleinrichtung, wenn sie mindestens ein Rückstellglied umfasst, welches sich direkt oder indirekt am mindestens einen Betätigungselement abstützt oder an diesem gehalten ist. Das Rückstellglied kann insbesondere in Form eines Federelements ausgebildet sein, welches als Druck- oder Zugglied gestaltet sein kann, um die Betätigungselemente entweder maximal aufeinander zu oder maximal voneinander weg zu halten, ohne dass eine Bedienperson eine Kraft auf das mindestens eine Betätigungselement ausüben muss. Das mindestens eine Rückstellglied kann beispielsweise mit einem Befestigungselement in Form einer Klammer oder einer Schraube am mindestens einen Betätigungselement festgelegt oder befestigt sein. Alternativ oder zusätzlich kann das mindestens eine Rückstellglied auch kraft- oder formschlüssig durch Klemmung am mindestens einen Betätigungselement befestigt werden kann, beispielsweise kann ein freies Ende des mindestens einen Rückstellglieds oder ein anderer Teil desselben in eine Ausnehmung oder eine Hinterschneidung am mindestens einen Betätigungselement formschlüssig oder im Wesentlichen formschlüssig eingreifen und/oder darin kraftschlüssig klemmend gehalten sein.

Vorteilhaft ist es, wenn das Instrument in Form eines Schiebeschaftinstruments ausgebildet ist mit einem zwei aneinander verschiebbar gelagerte Schaftteile umfassenden Schiebeschaft. Mit einem solchen Schiebeschaft lassen sich eine Vielzahl medizinischer Instrumente ausbilden, beispielsweise Klemmen, Scheren, Stanzen oder dergleichen.

Günstig ist es, wenn das mindestens eine Werkzeugelement an einem distalen Ende des Schiebeschafts angeordnet oder ausgebildet ist. Beispielsweise kann es sich dabei um ein Stanzwerkzeug handeln mit einer in distaler beziehungsweise proximaler Richtung verschiebbaren Schneide, die gegen ein entsprechend als Ambossglied ausgebildetes zweites Werkzeugelement bewegbar ist, um beispielsweise Knochen oder Gewebe zu bearbeiten.

Vorteilhaft ist es, wenn das Schiebeschaftinstrument in Form einer Knochenstanze ausgebildet ist. Mit einer solchen lassen sich insbesondere Knochen und andere Arten von Körpergewebe auf einfache und definierte Weise bearbeiten. Die nachfolgende Beschreibung bevorzugter Ausführungsformen der Erfindung dient im Zusammenhang mit den Zeichnungen der näheren Erläuterung. Es zeigen:
- Figur 1:: eine schematische perspektivische Gesamtansicht eines medizinischen Instruments in Form einer Knochenstanze;
- Figur 2:: eine teilweise durchbrochene Seitenansicht des in Figur 1 dargestellten Instruments vor der Montage eines Betätigungselements;

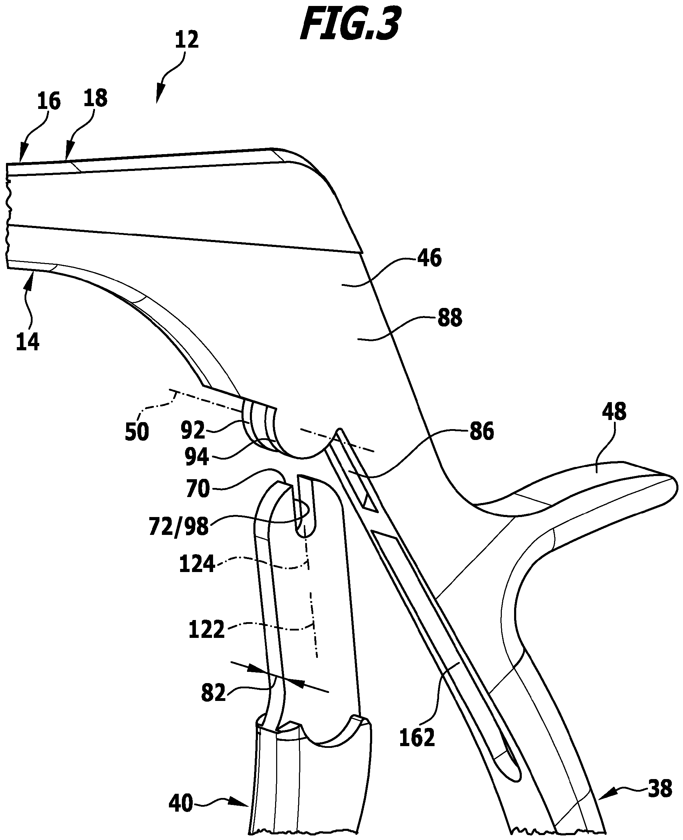

- Figur 3:: eine teilweise perspektivische Ansicht eines Teils des in Figur 2 dargestellten Instruments von der Gegenseite;
- Figur 4:: eine teilweise durchbrochene Ansicht eines Teils des Instruments mit in eine Lagerwellenaufnahme desselben eingeführter Lagerwelle des Betätigungselements in der Montagestellung;
- Figur 5:: eine teilweise durchbrochene Ansicht eines Teils des Instruments in der Arbeitsstellung mit maximal voneinander weg verschwenkten Betätigungselementen; und

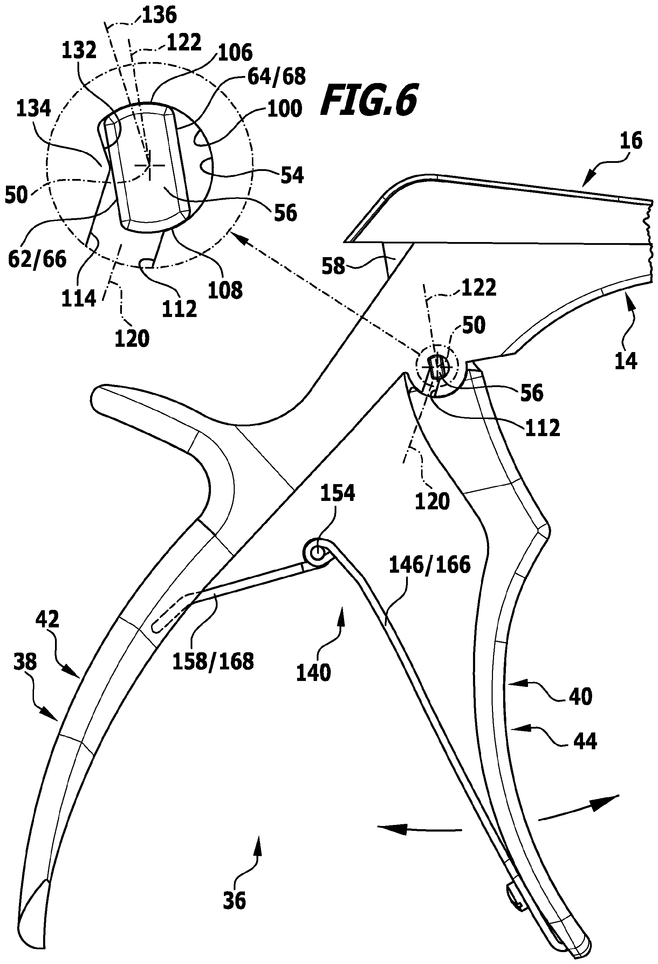

- Figur 6:: eine Seitenansicht des Instruments in einer Arbeitsstellung mit etwas aufeinander zu verschwenkten Betätigungselementen.

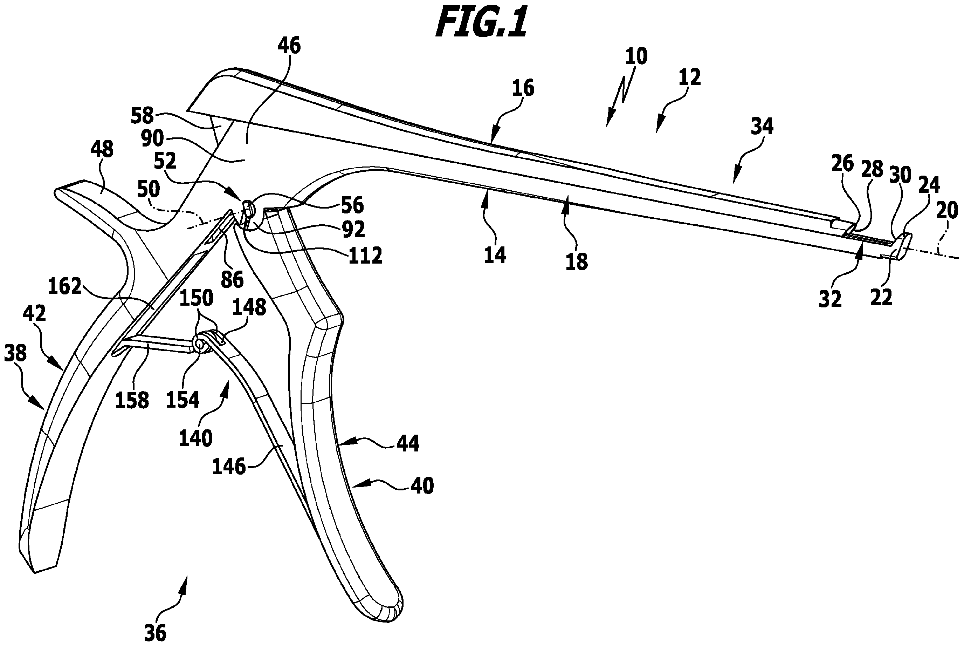

In Figur 1 ist schematisch ein medizinisches Instrument dargestellt und insgesamt mit dem Bezugszeichen 10 bezeichnet. Es ist in Form eines Schiebeschaftinstruments mit einem zwei aneinander verschiebbar gelagerte Schaftteile 14 und 16 umfassenden Schiebeschaft 18 ausgebildet. Die Schaftteile 14 und 16 sind mit einer nicht näher dargestellten Führung parallel zur Längsachse 20 relativ zueinander verschiebbar aneinander gelagert.

Der untere Schaftteil 14 des Schiebeschafts 18 trägt an seinem distalen Ende einen nach oben weisenden, bezogen auf eine Längsachse 20 des Schiebeschafts 18 quer abstehenden Vorsprung 22, welcher einen Amboss 24 für eine mit diesem zusammenwirkende Schneide 26 bildet, welche an einem distalen Ende des oberen Schaftteils 16 angeordnet oder ausgebildet ist. Die Schneide 26 bildet ein erstes Werkzeugelement 28, welches mit dem ein zweites Werkzeugelement 30 bildenden Amboss 24 zusammen eine Stanzeinrichtung 32 des eine Knochenstanze 34 bildenden Instruments 10 definiert.

Das Instrument 10 umfasst ferner eine Betätigungseinrichtung 36 mit zwei relativ zueinander verschwenkbaren Branchen 38 und 40, die jeweils ein Betätigungselement 42 beziehungsweise 44 der Betätigungseinrichtung 36 bilden. Die Branche 38 ist von einem proximalen Ende quer zur Längsachse 20 vom unteren Schaftteil 14 abstehend ausgebildet. Insbesondere können das Betätigungselement 42 und der Schaftteil 14 einstückig ausgebildet sein. Von der Branche 38 steht eine in Form eines Vorsprungs ausgebildete, in proximaler Richtung weisende Daumenauflage ab. Insgesamt ist somit die Branche 38 unbeweglich am Instrument 10 angeordnet.

Die Branche 40 ist relativ zur Branche 38 um eine Schwenkachse 50 verschwenkbar gelagert. Zu diesem Zweck umfasst das Instrument 10 eine Schwenklagerung 52 mit einer Lagerwellenaufnahme 54 und einer in dieser um die Schwenkachse 50 verdreh- oder verschwenkbaren Lagerwelle 56. Die Lagerwelle 56 ist an einem plattenförmig ausgebildeten Kopplungsabschnitt 58 des Betätigungselements 44 auf einer Seitenfläche desselben abstehend ausgebildet. Sie ist aus einem zylindrischen Grundkörper 60 ausgebildet und weist zwei Abflachungen 62 und 64 auf, die parallel zueinander verlaufende Seitenflächen 66 und 68 definieren.

Der Kopplungsabschnitt 58 weist an seinem freien Ende 70 eine schlitzförmige Ausnehmung 72 auf, welche eine Breite 74 aufweist, welche einem Außendurchmesser 76 eines am Schaftteil 16 angeordneten oder ausgebildeten Kupplungszapfens 78 entspricht. Der zylindrische Kupplungszapfen 78 ist quer zur Längsachse 20 in einer auf den Schaftteil 14 hin geöffneten Aussparung 80 des Schaftteils 16 angeordnet. Das Ende 70 ist halbkreisförmig verrundet. Eine Breite der Aussparung 80 entspricht einer Dicke 82 des Kopplungsabschnitts 58, so dass dieser in der Aussparung 80 seitlich geführt ist.

Am Ende 46, also am Übergangsbereich zwischen dem Schaftteil 14 und dem Betätigungselement 42 ist ein Lagerungsabschnitt 84 ausgebildet, welcher eine Durchbrechung 86 umfasst. Die Durchbrechung 86 wird von seitlichen Wandbereichen 88 und 90 verschlossen und eröffnet den Lagerungsabschnitt 84 nach oben in Richtung auf die nach unten hin geöffnete Aussparung 80, nach proximal sowie nach unten. Von den Wandbereichen 88 und 90 stehen nach unten in Draufsicht von der Seite halbkreisförmige Lagerbacken 92 und 94 ab. Eine Breite der Durchbrechung 86 entspricht der Dicke 82 des Kopplungsabschnitts 58, dass dieser durch die Durchbrechung 86 eingeführt und der Kupplungszapfen 78 und die Ausnehmung 72, welche Koppelelemente 96 und 98 bilden, miteinander in Eingriff bringbar sind.

Die Lagerwellenaufnahme 54 ist am Lagerbacken 92 ausgebildet. Sie umfasst einen hohlzylindrischen Innenwandflächenabschnitt 100, welcher sich über einen Umfangswinkel 102 von etwa 210° erstreckt. Ein vom Innenwandflächenabschnitt 100 definierter Durchmesser 104 der Lagerwellenaufnahme entspricht einem von zwei diametral zur Schwenkachse 50 einander gegenüberliegenden zylindrischen Wandflächenabschnitten 106 und 108 der Lagerwelle 56 definierten Außendurchmesser 110.

Die Lagerwellenaufnahme 54 ist seitlich geöffnet, und zwar in einer Richtung quer zur Schwenkachse 50 durch eine Einführöffnung 112. Diese ist in Form eines die Lagerwellenaufnahme 54 einseitig öffnenden Schlitzes 114 ausgebildet. Er erstreckt sich in radialer Richtung von der Schwenkachse 50 weg. Der Schlitz 114 umfasst zwei aufeinander zu weisende Innenflächen 116 und 118, die parallel zueinander und zu einer die Schwenkachse 50 enthaltenden Ebene 120 verlaufen. Die Innenflächen 116 weisen einen Abstand voneinander auf, der einer Breite 170 des Schlitzes 114 entspricht. Die Breite 170 entspricht einem Abstand der Seitenflächen 66 und 68 voneinander. So kann die Lagerwelle 54 durch den Schlitz 114 in die Lagerwellenaufnahme 54 eingeschoben werden.

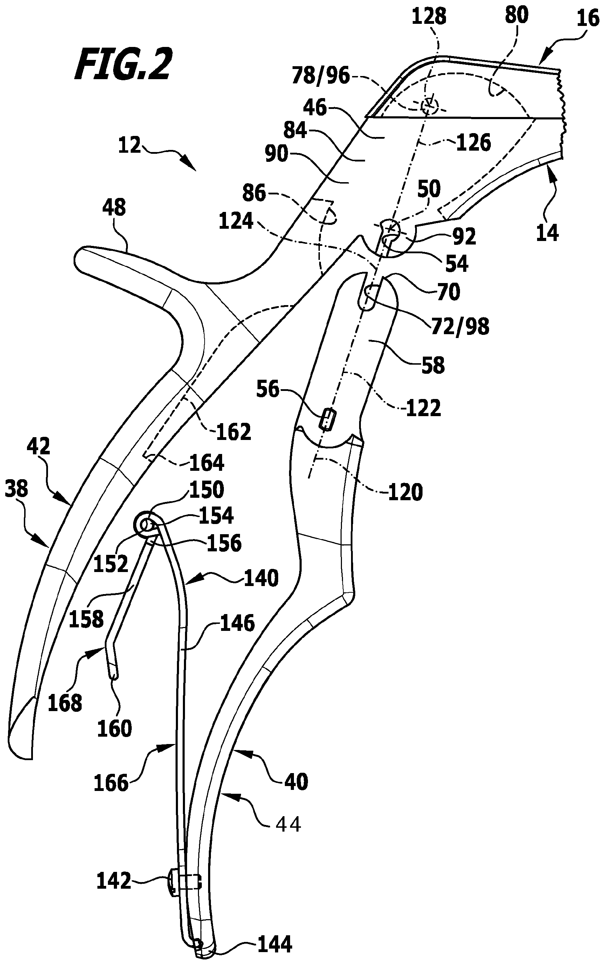

Die Lagerwelle 56 ist am Kopplungsabschnitt 58 derart angeordnet, dass sie eine Längsachse 122 definiert, die mit einer von der Ausnehmung 72 definierten Längsachse 124 zusammenfällt. Der Schlitz 114 definiert ebenfalls eine Längsachse 126, die eine Längsachse 128 des Kupplungszapfens 78 schneidet. Die beschriebene Ausrichtung der Längsachsen 122 und 124 sowie 126 und 128 ermöglicht es, den Kopplungsabschnitt 58 in einer Montagestellung, wie sie schematisch in Figur 2 dargestellt ist, mit dem Lagerungsabschnitt 84 in Eingriff zu bringen. In der Montagestellung werden die Längsachsen 122 und 126 parallel zueinander ausgerichtet, so dass die Lagerwelle 56 in die Lagerwellenaufnahme 54 durch den Schlitz 114 durch eine rein translatorische Bewegung einschiebbar und gleichzeitig der Kupplungszapfen 78 in die Ausnehmung 72 einführbar ist.

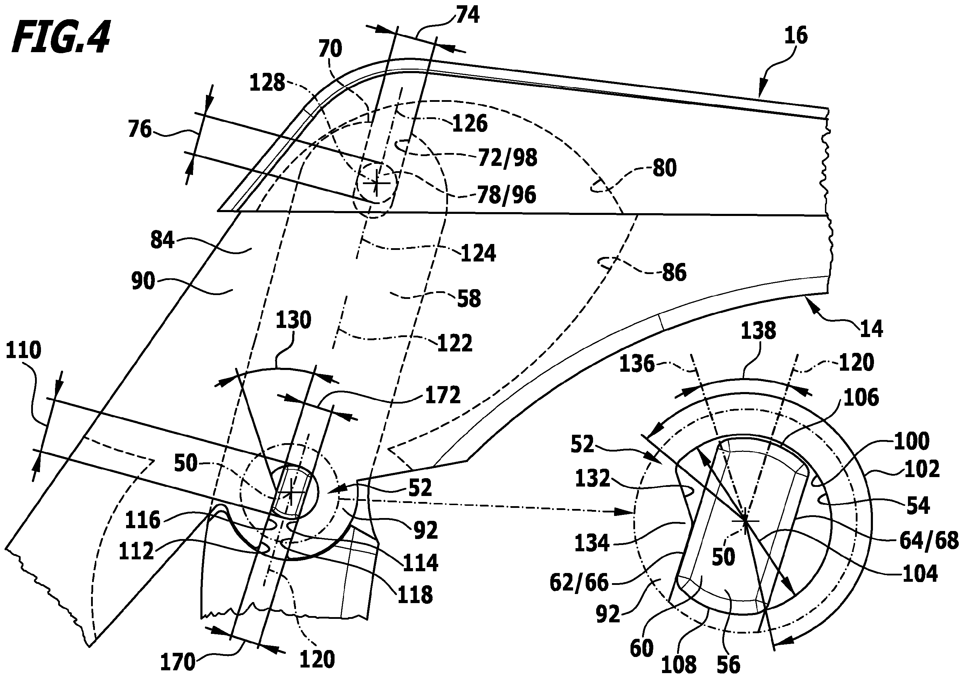

Ist die Lagerwelle 56 vollständig in die Lagerwellenaufnahme 54 eingeführt, wie dies schematisch in Figur 4 dargestellt ist, ist aufgrund der gewählten Maße von Durchmesser 104 und Außendurchmesser 110 eine Verschwenkung des Betätigungselements 44 um die Schwenkachse 50 möglich, und zwar um einen maximalen Verschwenkwinkel 130, welcher in der Montagestellung definiert wird zwischen der Seitenfläche 66 und einer Anschlagfläche 132 an einem an der Lagerwellenaufnahme 54 in Form eines Vorsprungs ausgebildeten Anschlag 134. Dieser ist ausgebildet zum Begrenzen einer Schwenkbewegung des Betätigungselements 44 um die Schwenkachse 50. Die Anschlagfläche 132 definiert eine Ebene, welche parallel zu einer weiteren, die Schwenkachse 50 enthaltenden Ebene 136 verläuft. Die Ebenen 120 und 136 definieren zwischen sich ebenfalls einen Verschwenkwinkel 138, welcher dem Verschwenkwinkel 130 entspricht.

Das Instrument 10 umfasst ferner eine Rückstelleinrichtung 140, entgegen deren Wirkung das Betätigungselement 44 in Richtung auf das Betätigungselement 42 hin verschwenkt werden kann. Die Rückstelleinrichtung 140 umfasst eine mit einer Schraube 142 an einem freien Ende 144 der Branche 40 befestigte Blattfeder 146, die an ihrem anderen freien Ende mit einem Schlitz 148 versehen ist, so dass zwei abstehende Vorsprünge 150 verbleiben, welche jeweils eingerollt sind und so jeweils eine Aufnahme 152 oder Öse für einen Lagerstift 154 bilden, welcher an einem freien Ende 156 eines zweiten Blattfederelements 158 ausgebildet ist und in die Aufnahmen 152 eingreift, so dass das Blattfederelement 158 und die Blattfeder 146 um eine vom Lagerstift 154 definierte Schwenkachse relativ zueinander verschwenkbar sind. Das freie Ende 144 der Blattfeder 146 kann alternativ oder zusätzlich auch kraft- oder formschlüssig durch Klemmung an der Branche 40 befestigt werden, beispielsweise kann das freie Ende 144 der Blattfeder 146 oder ein anderer Teil derselben in eine in den Figuren nicht näher dargestellte Ausnehmung oder eine Hinterschneidung an der Branche 40 formschlüssig oder im Wesentlichen formschlüssig eingreifen und/oder darin kraftschlüssig klemmend gehalten sein.

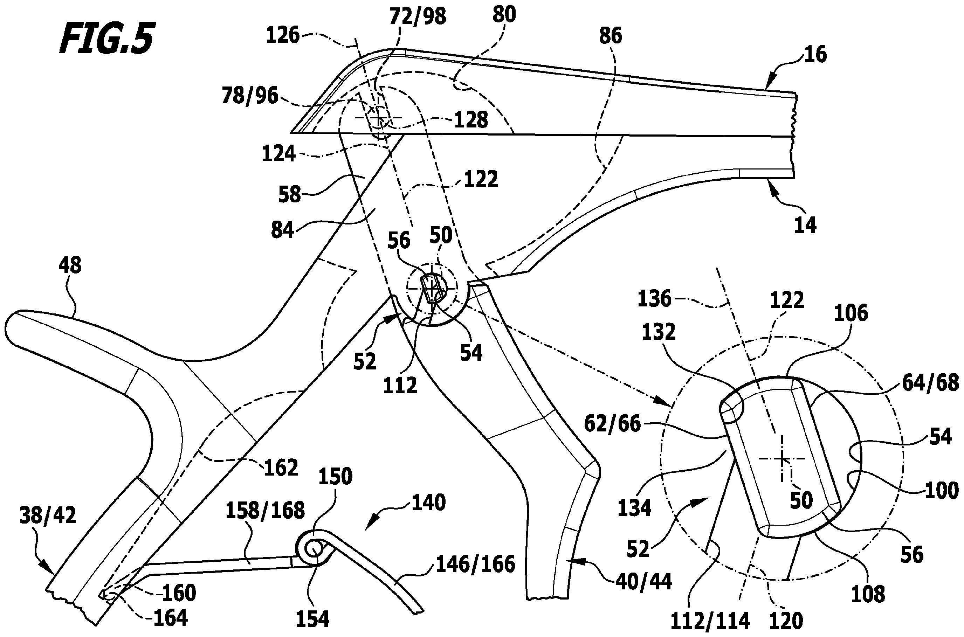

Das Blattfederelement 158 ist zu seinem anderen freien Ende 160 hin einmal schwach abgewinkelt und taucht in eine Ausnehmung 162 am Betätigungselement 42 ein. Die Ausnehmung 162 weist eine Anschlagfläche 164 auf, welche in Richtung auf das Schaftteil 16 hin weist. Das Ende 160 liegt in der Ausnehmung 162 an der Anschlagfläche 164 an. Die Rückstelleinrichtung 140 mit den jeweils ein Rückstellgliedern 166 und 168 bildenden Blattfeder 146 und Blattfederelement 158 hält die beiden Betätigungselemente 42 und 44 in einer schematisch in Figur 5 dargestellten Grundstellung, in welcher sie maximal voneinander weg verschwenkt sind. In dieser Grundstellung liegt die Abflachung 62 an der Anschlagfläche 132 an und begrenzt somit eine Bewegung der Branche 40 von der Branche 38 weg. Die Branche 40 kann nun entgegen der Wirkung der Rückstelleinrichtung 140 auf die Branche 38 hin verschwenkt werden. Solange der Verschwenkwinkel in der Arbeitsstellung, in welcher die Lagerwelle 56 in der Lagerwellenaufnahme 54 unverlierbar gehalten ist, kleiner ist als der Verschwenkwinkel 130, kann die Lagerwelle 56 nicht durch den Schlitz 114 aus der Lagerwellenaufnahme 54 austreten.

Die besondere Ausgestaltung der Schwenklagerung 52 ermöglicht eine einfache Montage des Instruments 10 ohne Werkzeuge. Die Lagerwelle 46 wird also nur in die Lagerwellenaufnahme 54 hineingesteckt. Die Schwenklagerung ist somit quasi schraubenlos.

Die oben in Verbindung mit einer Knochenstanze 34 beschriebene Schwenklagerung 52 ist prinzipiell auch bei jedem Handgriff eines medizinischen Instruments denkbar, der zwei relativ zueinander verschwenkbare Betätigungselemente umfasst. Insbesondere kann ein solcher Handgriff des Instruments beispielsweise mit einem Schaft desselben lösbar verbindbar sein, wie dies häufig bei endoskopischen Rohrschaftinstrumenten der Fall ist.

## Patentansprüche

1. Medizinisches Instrument (10) mit mindestens einem bewegbar angeordneten Werkzeugelement (28) und mindestens einem um eine Schwenkachse (50) verschwenkbar gelagerten, mit dem mindestens einen Werkzeugelement (28) direkt oder indirekt gekoppelten Betätigungselement (44) zum Bewegen des Werkzeugelements (28) infolge einer Verschwenkbewegung des Betätigungselements (44), wobei zur verschwenkbaren Lagerung des mindestens einen Betätigungselements (44) eine Schwenklagerung (52) umfassend eine Lagerwellenaufnahme (54) und eine in einer Arbeitsstellung in der Lagerwellenaufnahme (54) gehaltene Lagerwelle (56) vorgesehen ist, wobei das mindestens eine Betätigungselement (44) die Lagerwelle (56) oder die Lagerwellenaufnahme (54) umfasst, wobei die Lagerwellenaufnahme (54) eine Einführöffnung (112) umfasst und wobei die Lagerwelle (56) in einer Montagestellung in einer Richtung quer oder im Wesentlichen quer zur Schwenkachse (50) durch die Einführöffnung (112) in die Lagerwellenaufnahme (54) einführbar ist, wobei die Einführöffnung (112) in Form eines die Lagerwellenaufnahme (54) einseitig öffnenden Schlitzes (114) ausgebildet ist, wobei sich der Schlitz (114) in radialer Richtung von der Schwenkachse (50) weg erstreckt, wobei der Schlitz (114) eine Breite (170) aufweist, welche kleiner ist als ein von der Lagerwellenaufnahme (54) definierter Durchmesser (104), wobei die Lagerwelle (56) mindestens einen zylindrischen Wandflächenabschnitt (106, 108) umfasst, welcher konzentrisch zur Schwenkachse (50) ausgebildet ist, **dadurch gekennzeichnet, dass** die Lagerwelle (56) aus einem zylindrischen oder im Wesentlichen zylindrischen Grundkörper (60) gebildet ist und zwei von der Schwenkachse (50) beabstandete seitliche Abflachungen (62, 64) aufweist und dass die Lagerwelle (56) einstückig mit dem Instrument (10) oder dem mindestens einen Betätigungselement (44) ausgebildet ist.

2. Medizinisches Instrument nach Anspruch 1, **dadurch gekennzeichnet, dass** die in die Lagerwellenaufnahme (54) eingeführte Lagerwelle (56) durch Verschwenken um die Schwenkachse (50) von der Montagestellung in die Arbeitsstellung bringbar ist, in welcher die Lagerwelle (56) in der Lagerwellenaufnahme (54) unverlierbar gehalten ist.

3. Medizinisches Instrument nach Anspruch 1 oder 2, **dadurch gekennzeichnet, dass** der Schlitz (114) aufeinander zu weisende Innenflächen (116) umfasst.

4. Medizinisches Instrument nach Anspruch 3, **dadurch gekennzeichnet, dass** die Innenflächen (116) parallel oder im Wesentlichen parallel zu einer die Schwenkachse (50) enthaltenden Ebene (120) verlaufen.

5. Medizinisches Instrument nach einem der voranstehenden Ansprüche, **dadurch gekennzeichnet, dass** die Lagerwellenaufnahme (54) mindestens einen hohlzylindrischen Innenwandflächenabschnitt (100) umfasst, welcher konzentrisch zur Schwenkachse (50) ausgebildet ist.

6. Medizinisches Instrument nach einem der voranstehenden Ansprüche, **dadurch gekennzeichnet, dass** ein vom mindestens einen zylindrischen Wandflächenabschnitt (106, 108) definierter Außendurchmesser (110) der Lagerwelle (56) und ein vom mindestens einen Innenwandflächenabschnitt (100) definierter Innendurchmesser (104) der Lagerwellenaufnahme (54) aneinander angepasst sind für ein spielfreies oder im Wesentlichen spielfreies Verschwenken der Lagerwelle (56) in der Lagerwellenaufnahme (54).

7. Medizinisches Instrument nach einem der voranstehenden Ansprüche, **dadurch gekennzeichnet, dass** die Abflachungen (62, 64) parallel zu einander verlaufen.

8. Medizinisches Instrument nach einem der voranstehenden Ansprüche, **dadurch gekennzeichnet, dass** ein Abstand (172) der Abflachungen (62, 64) voneinander kleiner ist als eine Breite (170) der Einführöffnung (112).

9. Medizinisches Instrument nach einem der voranstehenden Ansprüche, **gekennzeichnet durch** eine Betätigungseinrichtung (36), welche das mindestens eine Betätigungselement (44) und ein zweites, am Instrument (10) unbeweglich angeordnetes Betätigungselement (42) umfasst.

10. Medizinisches Instrument nach einem der voranstehenden Ansprüche, **dadurch gekennzeichnet, dass** die Lagerwellenaufnahme (54) einen Anschlag (134) umfasst zum Begrenzen einer Schwenkbewegung des Betätigungselements (44) in einer Arbeitsstellung, in welcher die Lagerwelle (56) in der Lagerwellenaufnahme (54) unverlierbar gehalten ist.

11. Medizinisches Instrument nach Anspruch 10, **dadurch gekennzeichnet, dass** der Anschlag (134) in Form eines an der Lagerwellenaufnahme (54) ausgebildeten Vorsprungs ausgebildet ist und/oder dass der Anschlag (134) diejenige Stellung der Betätigungselemente (42, 44) definiert, in welcher sie maximal voneinander weg oder aufeinander zu verschwenkt sind, und/oder dass der Anschlag (134) eine Anschlagfläche (132) umfasst, an welcher eine Abflachung (62) der Lagerwelle (56) in derjenigen Stellung, in welcher die Betätigungselemente (42, 44) maximal voneinander weg oder aufeinander zu verschwenkt sind, mindestens teilweise flächig anliegt.

12. Medizinisches Instrument nach einem der voranstehenden Ansprüche, **dadurch gekennzeichnet, dass** das mindestens eine Betätigungselement (44) in Form einer verschwenkbaren Branche (40) ausgebildet ist, welche ein erstes Koppelelement (98) umfasst, welches mit einem zweiten Koppelelement (96), welches am mindestens einen Werkzeugelement (28) oder an einem mit diesem gekoppelten Kraftübertragungsglied (16) angeordnet oder ausgebildet ist, in einer Koppelstellung in Eingriff bringbar ist.

13. Medizinisches Instrument nach einem der voranstehenden Ansprüche, **gekennzeichnet durch** eine Rückstelleinrichtung (140) zum automatischen Überführen des mindestens einen Betätigungselements (44) von einer aus einer Grundstellung ausgelenkten Stellung zurück in die Grundstellung, wobei die Grundstellung diejenige Arbeitsstellung definiert, in welcher das mindestens eine Betätigungselement (44) gegenüber der Montagestellung um den größten Schwenkwinkel (130) gedreht ist.

14. Medizinisches Instrument nach Anspruch 13, **dadurch gekennzeichnet, dass** die Rückstelleinrichtung (140) mindestens ein Rückstellglied (166, 168) umfasst, welches sich direkt oder indirekt am mindestens einen Betätigungselement (42, 44) abstützt oder an diesem gehalten ist.

15. Medizinisches Instrument nach einem der voranstehenden Ansprüche, **dadurch gekennzeichnet, dass** das Instrument (10) in Form eines Schiebeschaftinstruments (12), insbesondere in Form einer Knochenstanze (34), ausgebildet ist mit einem zwei aneinander verschiebbar gelagerte Schaftteile (14, 16) umfassenden Schiebeschaft (18), wobei insbesondere das mindestens eine Werkzeugelement (28) an einem distalen Ende des Schiebeschafts (18) angeordnet oder ausgebildet ist.

## Claims

1. Medical instrument (10) comprising at least one moveable tool element (28) and at least one actuating element (44) which is mounted such as to be pivotal about a pivotal axis (50) and is directly or indirectly coupled to the at least one tool element (28) for the purposes of moving the tool element (28) as a result of a pivotal movement of the actuating element (44), wherein, for the pivotal mounting of the at least one actuating element (44), there is provided a pivotal bearing (52) comprising a bearing shaft holder (54) and a bearing shaft (56) which is held in the bearing shaft holder (54) in a working position, wherein the at least one actuating element (44) incorporates the bearing shaft (56) or the bearing shaft holder (54), wherein the bearing shaft holder (54) has an insertion opening (112) and wherein, in an assembly position, the bearing shaft (56) is insertable through the insertion opening (112) into the bearing shaft holder (54) in a direction that is transverse or substantially transverse to the pivotal axis (50), wherein the insertion opening (112) is in the form of a slot (114) opening the bearing shaft holder (54) on one side thereof, wherein the slot (114) extends away from the pivotal axis (50) in the radial direction, wherein the slot (114) has a width (170) which is smaller than a diameter (104) defined by the bearing shaft holder (54), wherein the bearing shaft (56) comprises at least one cylindrical wall surface section (106, 108) which is concentric with the pivotal axis (50), **characterized in that** the bearing shaft (56) is formed from a cylindrical or substantially cylindrical base body (60) and has two flattened portions (62, 64) which are laterally spaced from the pivotal axis (50) and **in that** the bearing shaft (56) is formed in one piece manner with the instrument (10) or the at least one actuating element (44).

2. Medical instrument in accordance with Claim 1, **characterized in that** the bearing shaft (56) that has been inserted into the bearing shaft holder (54) is moveable from the assembly position into the working position in which the bearing shaft (56) is held captive in the bearing shaft holder (54) by pivoting it about the pivotal axis (50).

3. Medical instrument in accordance with Claim 1 or 2, **characterized in that** the slot (114) comprises mutually facing inner surfaces (116).

4. A medical instrument in accordance with Claim 3, **characterized in that** the inner surfaces (116) run parallel or substantially parallel to a plane (120) containing the pivotal axis (50).

5. Medical instrument in accordance with any of the preceding Claims, **characterized in that** the bearing shaft holder (54) comprises at least one hollow cylindrical inner wall surface section (100) which is concentric with the pivotal axis (50).

6. Medical instrument in accordance with any of the preceding Claims, **characterized in that** an outer diameter (110) of the bearing shaft (56) which is defined by the at least one cylindrical wall surface section (106, 108), and an inner diameter (104) of the bearing shaft holder (54) which is defined by the at least one inner wall surface section (100) are mutually adapted for play-free or substantially play-free pivoting of the bearing shaft (56) in the bearing shaft holder (54).

7. Medical instrument in accordance with any of the preceding Claims, **characterized in that** the flattened portions (62, 64) run in parallel with each other.

8. Medical instrument in accordance with any of the preceding Claims, **characterized in that** a spacing (172) of the flattened portions (62, 64) from each other is smaller than the width (170) of the insertion opening (112).

9. Medical instrument in accordance with any of the preceding Claims, **characterized by** an actuating device (36) which comprises the at least one actuating element (44) and a second actuating element (42) which is arranged immovably on the instrument (10).

10. Medical instrument in accordance with any of the preceding Claims, **characterized in that** the bearing shaft holder (54) comprises a stop (134) for limiting a pivotal movement of the actuating element (44) in a working position in which the bearing shaft (56) is held captive in the bearing shaft holder (54).

11. Medical instrument in accordance with Claim 10, **characterized in that** the stop (134) is in the form of a projection that is formed on the bearing shaft holder (54) and/or **in that** the stop (134) defines that position of the actuating elements (42, 44) in which they are pivoted away from each other or towards one another to the maximum extent and/or **in that** the stop (134) comprises a stop surface (132) on which a flattened portion (62) of the bearing shaft (56) abuts flatly at least to a partial extent **in that** position in which the actuating elements (42, 44) are pivoted away from each other or towards one another to the maximum extent.

12. Medical instrument in accordance with any of the preceding Claims, **characterized in that** the at least one actuating element (44) is in the form of a pivotal branch (40) which comprises a first coupling element (98) which, in a coupling position, is moveable into engagement with a second coupling element (96) which is arranged or formed on the at least one tool element (28) or on a force transmission member (16) that is coupled thereto.

13. Medical instrument in accordance with any of the preceding Claims, **characterized by** a restoring device (140) for automatically transferring the at least one actuating element (44) from a position in which it is deflected from a basic position back into the basic position, wherein the basic position defines that working position in which the at least one actuating element (44) is rotated through the largest pivotal angle (130) in relation to the assembly position.

14. Medical instrument in accordance with Claim 13, **characterized in that** the restoring device (140) comprises at least one restoring member (166, 168) which is supported directly or indirectly on the at least one actuating element (42, 44) or is held thereon.

15. Medical instrument in accordance with any of the preceding Claims, **characterized in that** the instrument (10) is in the form of a sliding shaft instrument (12), in particular in the form of a bone punch (34), with a sliding shaft (18) comprising two mutually displaceable shaft parts (14, 16), wherein, in particular, the at least one tool element (28) is arranged or formed at a distal end of the sliding shaft (18).

## Revendications

1. Instrument médical (10) comprenant au moins un élément d'outil (28) agencé de manière mobile, et au moins un élément d'actionnement (44), qui est monté de manière à pouvoir pivoter autour d'un axe de pivotement (50), est couplé directement ou indirectement au dit au moins un élément d'outil (28), et est destiné à déplacer l'élément d'outil (28) en réponse à un mouvement de pivotement de l'élément d'actionnement (44), instrument médical
dans lequel pour le montage pivotant dudit au moins un élément d'actionnement (44), il est prévu un palier de pivotement (52) comportant un logement d'accueil d'arbre de palier (54), et un arbre de palier (56) maintenu dans le logement d'accueil d'arbre de palier (54), dans une position de travail,
dans lequel ledit au moins un élément d'actionnement (44) comporte l'arbre de palier (56) ou le logement d'accueil d'arbre de palier (54),
dans lequel le logement d'accueil d'arbre de palier (54) comporte une ouverture d'insertion (112), et dans lequel l'arbre de palier (56) peut être inséré, selon une direction transversale ou sensiblement transversale à l'axe de pivotement (50), dans le logement d'accueil d'arbre de palier (54), à travers l'ouverture d'insertion (112),
dans lequel l'ouverture d'insertion (112) est réalisée sous la forme d'une fente (114) ouvrant le logement d'accueil d'arbre de palier (54) sur un côté,
dans lequel la fente (114) s'étend de manière à s'éloigner dans la direction radiale, de l'axe de pivotement (50),
dans lequel la fente (114) présente une largeur (170), qui est inférieure à un diamètre (104) défini par le logement d'accueil d'arbre de palier (54), et
dans lequel l'arbre de palier (56) comporte au moins un secteur de surface de paroi cylindrique (106, 108), qui est réalisé concentriquement à l'axe de pivotement (50), **caractérisé en ce que** l'arbre de palier (56) est formé par un corps de base (60) cylindrique ou sensiblement cylindrique et présente deux méplats latéraux (62, 64) situés à distance de l'axe de pivotement (50), et **en ce que** l'arbre de palier (56) est réalisé d'un seul tenant avec l'instrument (10) ou ledit au moins un élément d'actionnement (44).

2. Instrument médical selon la revendication 1, **caractérisé en ce que** l'arbre de palier (56) inséré dans le logement d'accueil d'arbre de palier (54) peut être amené, par pivotement autour de l'axe de pivotement (50), de la position de montage à la position de travail dans laquelle l'arbre de palier (56) est maintenu de manière imperdable dans le logement d'accueil d'arbre de palier (54).

3. Instrument médical selon la revendication 1 ou la revendication 2, **caractérisé en ce que** la fente (114) comporte des surfaces intérieures (116) mutuellement en regard.

4. Instrument médical selon la revendication 3, **caractérisé en ce que** les surfaces intérieures (116) s'étendent parallèlement ou sensiblement parallèlement à un plan (120) contenant l'axe de pivotement (50).

5. Instrument médical selon l'une des revendications précédentes, **caractérisé en ce que** le logement d'accueil d'arbre de palier (54) présente au moins un secteur de surface de paroi intérieure cylindrique creux (100), qui est réalisé concentrique à l'axe de pivotement (50).

6. Instrument médical selon l'une des revendications précédentes, **caractérisé en ce qu'**un diamètre extérieur (110) de l'arbre de palier (56), qui est défini par ledit au moins un secteur de surface de paroi cylindrique (106, 108), et un diamètre intérieur (104) du logement d'accueil d'arbre de palier (54), qui est défini par ledit au moins un secteur de surface de paroi intérieure cylindrique creux (100), sont mutuellement adaptés l'un à l'autre pour assurer un pivotement sans jeu ou sensiblement sans jeu de l'arbre de palier (56) dans le logement d'accueil d'arbre de palier (54).

7. Instrument médical selon l'une des revendications précédentes, **caractérisé en ce que** les méplats (62, 64) s'étendent parallèlement l'un à l'autre.

8. Instrument médical selon l'une des revendications précédentes, **caractérisé en ce qu'**une distance d'espacement réciproque (172) des méplats (62, 64) est inférieure à une largeur (170) de l'ouverture d'insertion (112).

9. Instrument médical selon l'une des revendications précédentes, **caractérisé par** un dispositif d'actionnement (36), qui comprend ledit au moins un élément d'actionnement (44) et un deuxième élément d'actionnement (42) agencé de manière non mobile sur l'instrument (10).

10. Instrument médical selon l'une des revendications précédentes, **caractérisé en ce que** le logement d'accueil d'arbre de palier (54) comporte une butée (134) pour limiter un mouvement de pivotement de l'élément d'actionnement (44) dans une position de travail, dans laquelle l'arbre de palier (56) est maintenu de manière imperdable dans le logement d'accueil d'arbre de palier (54) .

11. Instrument médical selon la revendication 10, **caractérisé en ce que** la butée (134) est réalisée sous la forme d'une protubérance formée dans le logement d'accueil d'arbre de palier (54), et/ou **en ce que** la butée (134) définit la position des éléments d'actionnement (42, 44), dans laquelle ceux-ci sont pivotés de façon à être éloignés ou à être rapprochés au maximum l'un de l'autre, et/ou **en ce que** la butée (134) comporte une surface de butée (132) contre laquelle s'appuie, au moins en partie à plat en surface, un méplat (62) de l'arbre de palier (56), dans la position dans laquelle les éléments d'actionnement (42, 44) sont pivotés de façon à être éloignés ou à être rapprochés au maximum l'un de l'autre.

12. Instrument médical selon l'une des revendications précédentes, **caractérisé en ce que** ledit au moins un élément d'actionnement (44) est réalisé sous la forme d'une branche pivotante (40) comprenant un premier élément de couplage (98), qui, dans une position de couplage, peut être amené en prise avec un deuxième élément de couplage (96) agencé ou formé sur ledit au moins un élément d'outil (28) ou sur un organe de transmission de force (16) couplé à l'élément d'outil.

13. Instrument médical selon l'une des revendications précédentes, **caractérisé par** un dispositif de rappel (140) pour le transfert automatique dudit au moins un élément d'actionnement (44) d'une position déviée par rapport à une position de base, en retour à cette position de base, la position de base définissant la position de travail dans laquelle ledit au moins un élément d'actionnement (44) est tourné de l'angle de pivotement (130) le plus grand par rapport à la position de montage.

14. Instrument médical selon la revendication 13, **caractérisé en ce que** le dispositif de rappel (140) comprend au moins un organe de rappel (166, 168), qui s'appuie directement ou indirectement contre ledit au moins un élément d'actionnement (42, 44) ou bien est maintenu sur celui-ci.

15. Instrument médical selon l'une des revendications précédentes, **caractérisé en ce que** l'instrument (10) est réalisé sous la forme d'un instrument à corps de tige de coulissement (12), notamment sous la forme d'un poinçon à os (34), comprenant un corps de tige de coulissement (18) à deux parties de tige (14, 16) montées coulissantes l'une contre l'autre, en particulier ledit au moins un élément d'outil (28) étant agencé ou formé à une extrémité distale du corps de tige de coulissement (18) .
